# EUROPEAN PATENT APPLICATION

(11) **EP 2 108 311 A1**
(43) Date of publication of application: **14.10.2009**
(21) Application number: 08103478.7
(22) Date of filing: 10.04.2008
(51) Int. Cl.: A61B 5/024, A61B 5/22, A61B 5/00, A63B 21/00, A63B 24/00, A63B 71/06, G06F 19/00

(54) **A system for detecting and presenting heart rates of individuals during exercise**

(71) Applicant: Activio AB, 30122 Stockholm (SE)
(72) Inventor: Stolpe, Magnus, 112 48 Stockholm (SE); Magnergård, Tom, 113 26 Stockholm (SE); Magnergård, Daniel, 112 50 Stockholm (SE)
(74) Representative: Reyier, Ann-Mari

(57) **Abstract**

The present invention relates to a system for detecting and presenting heart rates of individuals during exercise. The system comprises: a plurality of body units (1) designed to be attached to the bodies of the individuals, comprising sensors for detecting the heart rate a receiver unit (3) for receiving information on heart rates from the body units during the exercise, a presentation unit (4) for presenting information on heart rates of the individuals, wherein the presentation unit is configured to display the heart rate information as a plurality of heart rate meters, and a central server unit (7) including a database (8) for storing information on the heart rates of individuals. The body unit is personal and comprises a memory for storing a unique user identity for identifying the owner of the body unit and the body unit is configured to transmit information on the user identity and the heart rate. The system further comprises at least one user terminal (2) configured to receive said user identities from the body units and to assign an individual heart rate meter to the received user identity, and at least one local computing unit (5) configured to receive information on the identity of the users and the assigned meters from the user terminal, to receive information on heart rates from said receiver, to send information on the heart rates together with the user identity to the central server unit, and to provide the presentation unit with information on the meters to be displayed and the present heart rates of the individuals exercising in the room.

## Description

### FIELD OF THE INVENTION

The present invention relates to a system for detecting and presenting heart rates of individuals during exercise, and more particularly to a system for detecting and presenting heart rates of individuals during group exercised performed simultaneously in a plurality of rooms of a training centre. The exercise, is for example, aerobics, workout or spinning.

### PRIOR ART

Apparatus for monitoring heart rates of individuals during exercise are known in the art. The Swedish patent Nr 526574 discloses as system for simultaneously monitoring heart rates of a plurality of exercising individuals. The system comprises an electrode belt to be attached to the body comprising a sensor for detecting heart rates and converting the detected heart rate into an electrical signal, and a transmitted for transmitting the signal. The system further comprises a first receiving unit to be attached to the body, a second receiving unit to be placed at a distance from the simultaneously monitoring heart rates of a plurality of exercising adapted for receiving signals from a plurality of first receiving units, and a central s unit for receiving and storing signals from the second receiving unit and for providing a presentation of the heart rates during exercise of the individuals. The first receiving unit comprising a receiver receiving the transmitted heart rate signals from the electrode belt, a converter for converting the heat rates into digital signals, and a transmitter for sending the digital heart rate signals to the second receiving unit. Such a system is suitable for supervising exercising bodies that are less than fifty and located at the same place, for example in the same room in a training centre. However, this system is not suitable for supervising a high number of training individuals, such as hundred and more training individuals, which are located in different places, for example in different rooms in the training centre.

A system for increasing motivation and making training more effective by displaying present heart rates of the individuals during training is known in the art. Such a system includes electrode belts designed to be attached to the bodies of the individuals. Each electrode belt comprises a sensor for detecting the heart rate and a transmitter for transmitting the heart rate. The electrode belts belong to the training centre and are borrowed by the individuals during a training session. Each electrode belts is provided with an identification number for identifying the electrode belt. Such a system further comprises a presentation unit, including a projector and a display screen on which the heart rate information is displayed as a plurality of individual heart rate meters. Each heart rate meter is provided with an identification number that corresponds to the identification number of the electrode belt. The system further comprises a computer including a database for storing information on heart rates during the training.

The system comprises a login terminal which logs the user in to the system before the training session begins. The user logs in to the system by manually feeding a username and/or a member number to the user terminal. Alternatively, the login terminal can be provided with a scanner and the user logs in by scanning the bar code on a personal member card. Further, the user must feed the identification number of the electrode belt to the login terminal so that the system will know which user is using which electron belt and the correct heat rate information is stored for the individual.

The computer with the database is located in connection to the training room and is provided with a receiver unit for receiving information on the heart rates from the body units during the training session. The computer stores the received heart rate information on the computer and also provides the presentation unit with heart rates to be presented on the heart rate meters. The displayed heart rate meters are provided with the same identification number as the body units and thereby it is possible for the user to identify which one of the meters displays his or her heart rate during the exercise. Thanks to the direct heart rate feedback, each exercising person is provided with a valuable tool in determining the correct extension level for optimizing results. Such a system is suitable for supervising a training class in one room of the training center. However, the system is not suitable for supervising a high number of individuals exercising simultaneously in a plurality of different rooms of the training centre. In particularly, it is time to consuming to distribute the electrode belts before each training session, to login the users to the system, and to collect the electrode belts after the training session has been ended.

### OBJECTS AND SUMMARY OF THE INVENTION

The object of the present invention is to provide an improved system for detecting and presenting heart rates of individuals during group exercise.

This object is achieved by a system as defined in claim 1.

According to the invention, the body units are personal and provided with a unique user identification identifying the owner of the body unit. The user identification is stored in a memory, which preferably is a persistent memory, of the body unit. This means that the user owns its own body unit and uses the same body unit during each exercise session. Accordingly, the training centre does no longer need to distribute the body units to the users before the training session or collect them after the training session is finished. A consequence of this is that the body unit is no longer associated with a certain heart rate meter. Therefore, the user has to be assigned an occasional heart rate meter during each training session.

According to the invention, the system comprises a user terminal configured to automatically receive information on the identities of the users when they pass by the user terminal. The user terminal is provided with a receiver configured to receive the information on the user identity from the body unit. When the user terminal receives and stores the user identity and the user is assigned a heart rate meter which is not yet occupied. According to one embodiment of the invention, the user terminal displays the available heart rate meters, the user selects one of the meters, and the user terminal assigns the selected meter to the user identity. It is an advantage if the user selects a heart rate meter, since it makes is easier to remember which one of the heat rate meters has been assigned to the user. Alternatively, the user terminal can automatically assigns a free heart rate meter to the user identity and displays the number of the allotted heart rate meter to the user.

The user terminal according to the invention makes it quick and easy to assign heart rate meters to the users. With a system according to the invention it is not necessary to manually enter any username or member number or to scan any bar code. Accordingly, queues are avoided.

Another object of the object of the present invention is to provide a system for detecting and presenting heart rates of individuals during group exercise performed simultaneously in a plurality of rooms of a training centre. According to an embodiment of the invention, at least one presentation unit is located in each of said rooms of the training centre, at least one heart rate receiver unit is located in each of said rooms for receiving information on heart rates from the body units is positioned in the room, at least one user terminal is located in connection with each of said rooms, and at least one of said local computing unit associated with each of said rooms and configured to receive information on the identity of the users and the assigned meters from the user terminal located in connection to the same room, to receive information on heart rates from said receiver located in the room, and to provide the presentation unit in the room with information on the meters to be displayed and the present heart rates of the individuals exercising in the room.

As each room can be provided with its own user terminal, preferably located in connection to the door of the room, many individuals can simultaneously be assigned heart rate meters.

In the prior art system, the heart rate information is transmitted directly to the central server unit and stored in the database. According to this embodiment of the invention, each room is provided with a receive unit for receiving the heart rate information from the body units and the heart rate information is temporarily stored in a local computer unit, preferably a local computer unit is arranged in or in connection with each of the rooms. The local computer unit may process the heart rate information and later, for example when the training session has ended, send the processed heart rate information to the central server unit. The local computer unit also provides the presentation unit with information on which heart rate meters has been assigned, and accordingly has to be displayed during the training session, and the present heart rates to be displayed on the meters of the individuals exercising in the room at the moment. Preferably, the heart rate data is transferred via the Internet to the central server unit and the heart rate data is stored in the database together with the user identity. The central server unit may store heart rate data from a plurality of training centers at different locations.

This embodiment enables individuals to simultaneously train in different locations in a training centre, or at different training centers, and ensures training feedback to be saved for post exercise analysis. The feedback makes it easier for everyone to find the right intensity during the training session. All the data is saved in a personal database as a tool to follow up on sessions later on. Further, this embodiment makes it possible to supervise heart rates of more than two hundred individuals exercising simultaneously.

According to an embodiment of the invention, the body unit comprises a transmitter configured to transmit information on the user identity, i.e. the user identity, together with the heart rate information. As the user identity is transmitted together with the heart rate information by the same transmitter no extra hardware is needed on the body unit and thereby no extra cost is added to the body unit.

According to an embodiment of the invention, the user terminal is configured to receive the use identity when the body unit is positioned close to the user terminal, preferably at a distance from the user terminal which is less than 1 m. This embodiment enables a line of users to quickly pass by the user terminal.

According to an embodiment of the invention, the body unit comprises a transmitter configured to transmit said user identity information by means of a radio signal. The transmitter can either be active or passive. An example of such a transmitter is RFID (Radio Frequency Identification).

Further developments of the device are characterized by the features of the additional claims.

### BRIEF DESCRIPTION OF THE DRAWINGS

The invention will now be explained more closely by the description of different embodiments of the invention and with reference to the appended figures.

Fig. 1 shows a block diagram of a system for detecting and presenting heart rates according to an embodiment of the invention.

Fig. 2 shows an example of how a local part of the system can be implemented in a training centre with two rooms.

Fig. 3 shows an example of a view including a plurality of heart rate meters presented for a training class.

Figure 4 shows the body unit in more detail.

### DETAILED DESCRIPTION OF PREFERRED EMBODIMENTS OF THE INVENTION

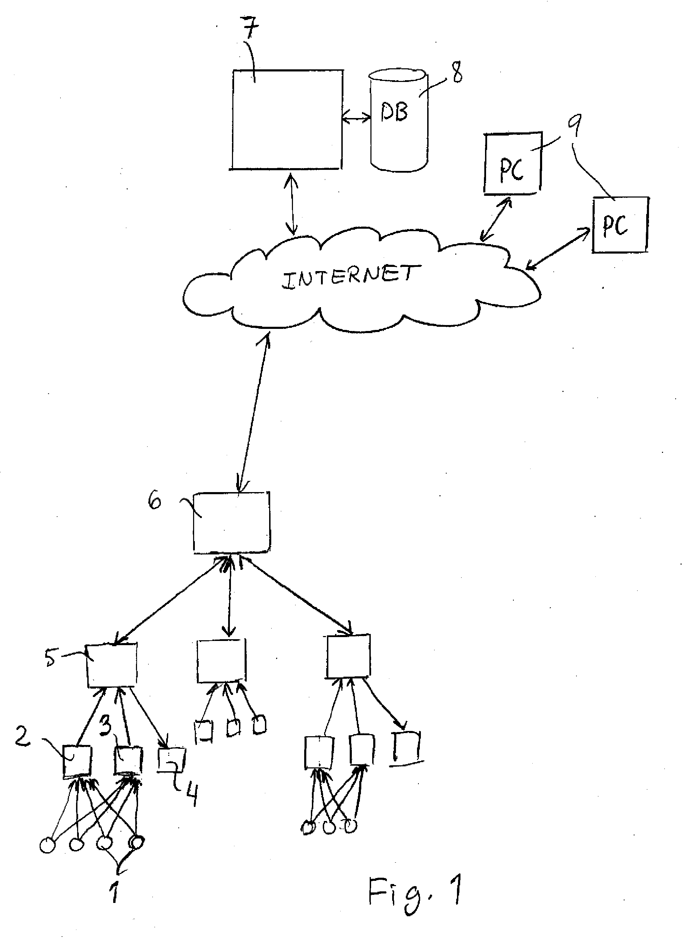

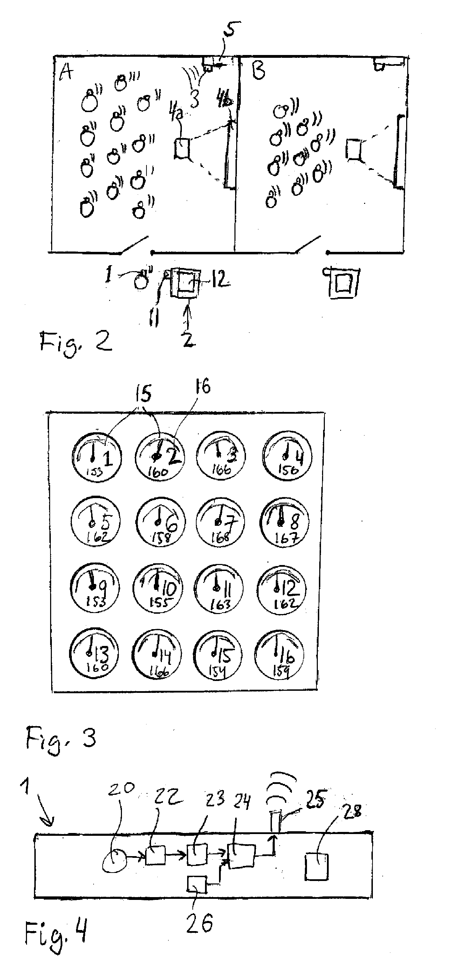

Figure 1 shows an example of a system according to the invention for detecting and presenting heart rates of individuals during exercise. The system comprises a plurality of body units 1 designed to be attached to the bodies of the individuals. For example, the body unit is designed as an electrode belt for heart rate monitoring and is to be worn around the chest. Each body unit comprises a sensor for detecting the heart rate, for example electrodes with ECG-accuracy, and the heart rate information is wirelessly transmitted by means of a transmitter. The transmitter sends a radio signal at every heart beat. The body unit will be described in more detail with reference to figure 4. Each body unit is individual and is provided with a unique user identity for identifying the owner of the body unit. The body unit is configured to transmit the user identity together with the sensed heart rates.

The system further comprises a plurality of user terminals 2 configured to automatically receive the user identities and enter the user identities to the system when the users, wearing the body units, pass the user terminal. The user terminal is configured to receive the information on the user identity from the body units. The user terminal is also configured to assign an individual heart rate meter to each user identity. This is, for example, done either by displaying a number of free heart rate meters with their unique numbers to the user and the user selects one of the free meters. For example, all meters belonging to the room is displayed and the free meters, open for selection, are high lighted. Alternatively, the user terminal automatically assigns a free heart rate meter to the user and displays the number of the assigned heart rate meter. The number of the assigned heart rate meter is stored together with the user identity. The user terminals comprises a processing unit, such as a CPU, suitable memory, a display device, a receiver for receiving the user identities, and communication means for communicating with the local computer unit.

The system further comprises a plurality of heart rate receiver units 3, wherein at least one heart rate receiver unit is located in each room for receiving information on heart rates from all body units positioned in the room.

The system further comprises a plurality of presentation units 4. At least one presentation unit is located in each of the rooms of the training centre. The presentation unit is configured to present information on present heart rates of the individuals during the training session. The presentation unit displays the heart rate information as a plurality of heart rate meters. Each individual is assigned its own heart rate meter. Each heart rate meter is provided with a unique number which makes it possible for the user to identify his own personal meter. The presentation unit includes a projector and a display screen.

The system further comprises a plurality of local computing units 5 configured to communicate with the user terminal 2, the heart rate receiving unit 3 and the presentation unit 4 of the room. Preferably, one local computer unit is located in or in connection to each training room of the training centre. The local computing unit 5 is configured to receive information on the identities of the users and the assigned meters from the user terminal 2, to continuously receive information on the present heart rates from the heart rate receiving unit 3 located in the room, and to provide the presentation unit 4 with information on the heart rate meters to be displayed and the present heart rates of the individuals exercising in the room. Further, the local computing unit is configured to communicate with a common computer unit 6, which is configured to communicate with all of the local computing units 5 of the training centre. Each of the local computing units 5 is configured to send information on the heart rates together with the user identity to the common computer unit 6. The common computer unit 6 handles all the heart rate information of the training centre and is configured to communicate with a central server unit 7, for example via the Internet. This embodiment makes it possible to reach and track data via the internet.

The central server unit 7 includes a database 8 for storing heart rate information of the individuals. Each individual has a personal part of the database reachable via the user identification and includes personal heart rate data. The registered users are enabled to access the heart rate data via a web interface after exercise, for example, by means of personal computers 9.

The common computer unit 6 is optional. In an alternative embodiment the local communication units 5 may communicate directly with the central server unit 7. Upon receiving the user identity, it is also possible that the local computer retrieves data from the database 8 of the central server, such as maximum heart rate, which information can be displayed on the presentation unit during the training session.

Figure 2 shows two rooms A and B of a training centre designed for hosting a plurality of exercising persons during a training session. Each of the training persons is carrying a body unit 1 including a transmitter for wirelessly transmitting information on heart rates and the identity of the user. Each of the rooms is provided with a user terminal 2 located outside the rooms, preferably in vicinity of the entrance to the room. The user terminal 2 is provided with a receiver 11 for receiving the user identity from the body unit and a display unit 12 for displaying information of available heart rate meters, or for displaying information on an automatically assigned heart rate meter.

Each room is provided with a presentation unit including a projector 4a and a display screen 4b. The presentation unit is also provided with means for creating a graphical presentation of the heart rate meters. The room is further provided with a local computer 5 configured to provide the presentation unit with all necessary information of the presentation. In this embodiment the local computing unit 5 includes a receiver unit 3 for receiving information on the heart rates from the body units located in the room during the training session. Alternatively, the receiver unit 3 can be located at a distance from the computing unit 5. It is also possible to arrange the local computing unit 5 outside the room and to arrange the receiver unit 3 inside the room.

Figure 3 shows an example of a view presented on the screen 4b during the training session. Each person training in the room has been assigned an individual heart rate meter 15. Each heart rate meter is provided with an ID number 16 so that the user knows which of the heart rate meters shows his or her heart rate. The heart rate meter also displays an indicator showing the current heart rate. Optionally, a percentage of estimated maximum heart rate can also be displayed.

Figure 4 shows the body unit 1 in more detail. The body unit comprises a sensor 20 for detecting heart beats and generating a heart rate signal, and a converter 22 for converting the heart rate signal from the sensor into digital signals. The body unit 1 further comprises a computer 23, a transmitter 24 and an antenna 25. The computer 23 is preferably a micro controller having a processor and a memory. The transmitter 24 includes a radio module and sends a radio signal at every heart beat. The transmitter is activated when the user attaches the body unit to his body. More particularly, the transmitter is activated when the sensor starts to detect the heart beats. The transmitter is deactivated when the user takes off the body unit, more particularly when the sensor can not detect any heart beats, or at least a few minutes thereafter.

The body unit further comprises a memory 26 for storing the unique user identity. The memory 26 is, for example, a non volatile memory. The digital signals from the converter 22 are combined with the user identity from the memory 26 into a data package. The body unit also comprises a battery 28. The transmitter 24 and the antenna 25 are adapted for wirelessly transmitting radio signals at a distance of at least 20 meters. The heart rate receiver unit 3 located in the training room is adapted for receiving the signals from the body unit 1. The heart rate receiving unit 3 comprises a radio module, which is similar to the radio module of the transmitter 24. The local computing unit 5 comprises a communicating unit adapted for communicating with the presentation unit 4a. The body unit is configured to automatically send heart rate information together with user identity information upon detecting a heart beat. This means that it is not necessary to turn on or off the body unit.

The heart rate data is wirelessly transmitted continuously from each body unit in real time. During the exercise, every participant is given a visual feedback on the training. This makes it easier for everyone to find the right intensity during the training session. All the data is saved in a personal database as a tool to follow up the sessions later on. Even if the training facilities are widespread, the data are collected and easily connected to wherever members or users are located. Before entering the class, the users enter the system with user identification. Together with the user identification data from the training session will automatically be transmitted and saved in the personal database. According to an embodiment of the invention, participants are enabled to access the heart rate data via a web interface after exercise. The information is reachable whenever or wherever the user is in need of the information. All the personal data is protected with a secure login so that only the right person gets access.

The system according to the invention works as a full health club solution for heart rate monitoring, and serves several training areas within the training centre and automatically transfers data to be organized in a database. Exercise can be done in varies facilities and classes no matter of time and distance keeping them apart, the results are still safely kept only at the hands of the individuals.

The present invention is not limited to the embodiments disclosed but may be varied and modified within the scope of the following claims. For example, the body unit can be provided with two transmitters, one for transmitting heart rates and one for transmitting the user identification. In this case, the transmitter for transmitting the user identification can be a RFID.

It is also possible that two neighboring rooms of the training center share one or more units, such as a receiving unit, a user terminal and/or a local computing unit.

## Claims

1. A system for detecting and presenting heart rates of individuals during exercise, wherein the system comprises:
a plurality of body units (1) designed to be attached to the bodies of the individuals, each body unit comprising a sensor (20) for detecting the heart rate and is configured to transmit information on the heart rate,
a receiver unit (3) for receiving said information on heart rates from the body units during the exercise,
a presentation unit (4,4a-b) for presenting information on heart rates of the individuals, wherein the presentation unit is configured to display the heart rate information as a plurality of heart rate meters (15), and
a central server unit (7) including a database (8) for storing information on the heart rates of individuals, **characterized in that** the body unit is personal and comprises a memory (26) for storing a unique user identity for identifying the owner of the body unit and the body unit is configured to transmit information on the user identity, and the system further comprises:
at least one user terminal (2) configured to receive said user identities from the body units when the body unit is positioned close to the user terminal and to assign an individual heart rate meter to the received user identity, and
at least one local computing unit (5) configured to receive information on the identity of the users and the assigned meters from the user terminal, to receive information on heart rates from said receiver, to send information on the heart rates together with the user identity to the central server unit, and to provide the presentation unit with information on the meters to be displayed and the present heart rates of the individuals exercising in the room.

2. The system according to claim 1, wherein the system is configured to detect and present heart rates of individuals during group exercise performed simultaneously in at least two rooms of a training center, and
at least one of said presentation unit is located in each of said rooms,
at least one of said heart rate receiver unit is located in each of said rooms for receiving information on heart rates from the body units positioned in the room,
at least one of said user terminal (2) is located in connection with each of said rooms, and
at least one of said local computing unit is associated with each of said rooms and configured to receive information on the identity of the users and the assigned meters from the user terminal located in connection to the same room, to receive information on heart rates from said receiver located in the room, and to provide the presentation unit in the room with information on the meters to be displayed and the present heart rates of the individuals exercising in the room.

3. The system according to claim 1 or 2, wherein the body unit comprises a transmitter (24,25) configured to transmit information on the user identity together with the heart rate information.

4. The system according to any of the previous claims, wherein each user terminal comprises an identity receiver unit (11) configured to receive said user identity and to register the user identity when the body unit is positioned close to the user terminal (2).

5. The system according to claim 4, wherein said identity receiver unit (11) is configured to receive said user identity when the body unit is at a distance from the user terminal which is less than 1 m.

6. The system according to any of the previous claims, wherein the body unit comprises a transmitter (24,25) configured to transmit said user identity information by means of a radio signal.

7. The system according to any of the previous claims, wherein said user terminal (2) comprises a display unit (12) and the user terminal is configured, upon receiving a user identity, to display which heart rate meters are free to select, to receive information on a heart rate meter selected by the user, and to assign the selected heart rate meter to the user identity.
